(19) Europäisches Patentamt
European Patent Office
Office européen des brevets

(11) Publication number: 0 038 135 B1

(12) **EUROPEAN PATENT SPECIFICATION**

(45) Date of publication of patent specification: 11.07.84

(51) Int. Cl.³: C 07 C 103/52, A 61 K 37/43

(21) Application number: 81301357.0

(22) Date of filing: 27.03.81

# (54) LRF antagonists.

(30) Priority: 15.04.80 US 140487
08.05.80 US 147555
29.08.80 US 182594

(43) Date of publication of application:
21.10.81 Bulletin 81/42

(45) Publication of the grant of the patent:
11.07.84 Bulletin 84/28

(84) Designated Contracting States:
AT BE CH DE FR GB IT LI LU NL SE

(56) References cited:
GB - A - 1 532 211
GB - A - 2 009 182
US - A - 4 087 416

CHEMICAL ABSTRACTS, vol. 86, no. 21, May 23, 1977, page 502, abstract 155951k, Columbus, Ohio, US. K. NIKOLICS et al.: "Synthesis and biological activity of position 1 analogs of LH-RH"

(73) Proprietor: THE SALK INSTITUTE FOR BIOLOGICAL STUDIES
10010 North Torrey Pines Road
La Jolla California 92038 (US)

(72) Inventor: Rivier, Catherine Laure
9674 Blackgold Road
La Jolla California (US)
Inventor: Rivier, Jean Edouard Frederic
9674 Blackgold Road
La Jolla California (US)
Inventor: Vale, Wylie Walker, Jr.
1643 Valdez
La Jolla California (US)

(74) Representative: Lawrence, Malcolm Graham et al,
BROOKES & MARTIN High Holborn House 52/54
High Holborn
London WC1V 6SE (GB)

(56) References cited:
CHEMICAL ABSTRACTS, vol. 78, no. 19, May 14, 1973, page 513, abstract 124875u, Columbus, Ohio, US; N. YANAIHARA et al.: "Syntheses and biological activities of analogs of luteinizing hormonereleasing hormone (LH-RH) substituted in position 1 or 2"

EP 0 038 135 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to peptides which inhibit the release of gonadotropins by the pituitary gland in mammalians, including humans and to methods of preventing ovulation and/or inhibiting the release of steroids. More particularly, the present invention is directed to peptides which inhibit gonadal function and the release of the steroidal hormones, progesterone and testosterone.

The pituitary gland is attached by a stalk to the region in the base of the brain known as the hypothalamus. The pituitary gland has two lobes, the anterior and the posterior lobes. The posterior lobe of the pituitary gland stores and passes onto the general circulation two hormones manufactured in the hypothalamus, these being vasopressin and oxytocin. The anterior lobe of the pituitary gland secretes a number of hormones, which are complex protein or glyco-protein molecules that travel through the bloodstream to various organs and which, in turn, stimulate the secretion into the blood stream of other hormones from the peripheral organs. In particular, follicle stimulating hormone (FSH) and luteinizing hormone (LH), sometimes referred to as gonadotropins or gonadotropic hormones, are released by the pituitary gland. These hormones, in combination, regulate the functioning of the gonads to produce testosterone in the testes and progesterone and estrogen in the ovaries, and also regulate the production and maturation of gametes.

The release of a hormone by the anterior lobe of the pituitary gland usually requires a prior release of another class of hormones produced by the hypothalamus. One of the hypothalamic hormones acts as a factor that triggers the release of the gonadotropic hormones, particularly LH. The hypothalamic hormone which acts as a releasing factor for LH is referred to herein as LRF although it has also been referred to as LH-RH and as GnRH. LRF has been isolated and characterized as a decapeptide having the following structure:

$$\text{p-Glu-His-Trp-Ser-Tyr-Gly-Leu-Arg-Pro-Gly-NH}_2$$

Peptides are compounds which contain two or more amino acids in which the carboxyl group of one acid is linked to the amino group of the other acid. The formula for LRF, as represented above, is in accordance with conventional representation of peptides where the amino group appears to the left and the carboxyl group to the right. The position of the amino acid residue is identified by numbering the amino acid residues from left to right. In the case of LRF, the hydroxyl portion of the carboxyl group of glycine has been replaced with an amino group ($NH_2$). The abbreviations for the individual amino acid residues above are conventional and are based on the trivial name of the amino acid: where p-Glu is pyroglutamic acid, His is histidine, Trp is tryptophan, Ser is serine, Tyr is tyrosine, Gly is glycine, Leu is Leucine, Arg is arginine and Pro is proline. Except for glycine, amino acids of the peptides of the invention are of the L-configuration unless noted otherwise.

It is known that the substitution of D-amino acids for Gly in the 6-position of the LRF decapeptide provides a peptide material having from about 1 to 35 times greater potency than does LRF to effect the release of LH and other gonadotropins by the pituitary gland of mammalians. The releasing effect is obtained when the LRF analog is administered to a mammalian intravenously, subcutaneously, intramuscularly, orally, intranasally or intravaginally.

It is also known that substitution of various amino acids for His (or the deletion of His) at the 2-position of the LRF decapeptide produces analogs having an inhibitory effect on the release of LH and other gonadotropins by the pituitary gland of mammalians. In particular, varying degrees of inhibition of the release of LH are obtained when His is deleted (des His) or replaced by D-Ala, D-Phe or Gly. The inhibitory effect of such peptides modified at the 2-position can be further enhanced when a D-amino acid is substituted for Gly in the 6-position of the decapeptides. For example, the peptide: p-Glu-Trp-Ser-Tyr-D-Ala-Leu-Arg-Pro-Gly-$NH_2$ is 4 times more potent as an inhibitor for the release of gonadotropins than is the same peptide where Gly is present in the 6-position rather than D-Ala.

Our UK Application No. 78 46680 (2 009 182A) discloses peptides of the formula:—

$$R_1\text{-}R_2\text{-}R_3\text{-Ser-Tyr-}R_4\text{-}R_5\text{-Arg-}R_6$$

in which $R_1$ is, for example, D-Pro; $R_2$ is, for example, D-Phe, D-His, D-Trp or Trp; $R_3$ is, for example, D-Trp, Trp, D-Phe or D-His; $R_4$ is Gly, D-Trp, D-Phe or D-Tyr; $R_5$ is Leu or N$\alpha$Me-Leu and $R_6$ is Pro-Gly-$NH_2$ or Pro-NH-$CH_2$-$CH_3$.

Some female mammalians who have no ovulatory cycle and who show no pituitary or ovarian defect begin to secrete normal amounts of the gonadotropins LH and FSH after the appropriate administration of LRF. Thus, the administration of LRF is considered suitable for the treatment of those cases of infertility where a functional defect resides in the hypothalamus.

There are also reasons for desiring to prevent ovulation in female mammalians, and the administration of LRF analogs that are antagonistic to the normal function of LRF have been used to prevent ovulation. For this reason, analogs of LRF which are antagonistic to LRF are being investigated for their potential use as a contraceptive or for regulating conception periods. It is desired to provide

peptides which are strongly antagonistic to endogenous LRF and which prevent secretion of LH and the release of steroids by the gonads of mammals.

The present invention provides peptides which inhibit the release of gonadotropins in mammalians, including humans, and also provides methods for inhibiting the release of steroids by the gonads of male and female mammalians. The improved LRF analogs are antagonistic to LEF and have an inhibitory effect on the reproduction processes of mammalians. These analogs may be used to inhibit the production of gonadotropins and sex hormones under various circumstances including precocious puberty, hormone dependent neoplasia, dysmenorrhea and endometriosis. The LRF antagonists of the invention, although having structural similarity to the peptides of our Application No. 7846680 (2 009 182A), are markedly more effective in use.

Generally, in accordance with the present invention, peptides have been synthesized which strongly inhibit the secretion of gonadotropins by the pituitary gland of mammalians, including humans, and/or inhibit the release of steroids by the gonads. These peptides are analogs of LRF wherein there is a 1-position substitution in the form of either dehydroproline or meta-thiazolidine-2-carboxylic acid, and preferably substituents are also present at the 2-, 3- and 6-positions. The 1-position substituent may be modified so that its alpha amino group contains an acyl group, such as formyl, acetyl, acrylyl, vinylacetyl, or benzoyl. Dehydro L-Pro is preferred in the 1-position. Modified D-Phe is preferably present in the 2-position and provides increased antagonistic activity as a result of the specific benzene ring. Single substitutions for hydrogen are preferably made in the para- or 4-position, and double substitutions are made preferably in the 2,4- or the 3,4-positions. The substitutions are most preferably selected from dichloro, methyl, fluoro, difluoro, trifluoromethyl, methoxy, bromo, dibromo, nitro, dinitro, acetylamino and methyl mercapto. D-Trp is preferred in the 3-position, and imBzl D-His or D-Trp or some other lipophilic aromatic D-amino acid is preferred in the 6-position, although Gly or any D-isomer amino acid, e.g. D-Leu and D-Ser(O-t But), may be used. The substitutions in the 7- and 10-positions are optional.

Because these peptides are highly potent to inhibit release of LH, they are often referred to as LRF antagonists. The peptides inhibit ovulation of female mammals when administered at very low levels at proestrous and are also effective to cause resorption of fertilized eggs if administered shortly after conception.

More specifically, the peptides of the present invention are represented by the following formula:

$$X\text{-}R_1\text{-}R_2\text{-}R_3\text{-Ser-Tyr-}R_4\text{-}R_5\text{-Arg-Pro-}R_6$$

wherein X is hydrogen or an acyl group having 7 or less carbon atoms; $R_1$ is dehydro Pro, dehydro D-Pro, Thz or D-Thz; $R_2$ is D-Phe, D-His, D-Trp, Trp, Cl-D-Phe, dichloro-D-Phe, $CF_3$-D-Phe, F-D-Phe, difluoro-D-Phe, AcNH-D-Phe, $NO_2$-D-Phe, dinitro-D-Phe, Br-D-Phe, dibromo-D-Phe, $CH_3S$-D-Phe, $OCH_3$-D-Phe or $CH_3$-D-Phe; $R_3$ is D-Trp, Trp, D-Phe or D-His; $R_4$ is Gly or a D-isomer amino acid; $R_5$ is Leu or $N^{\alpha}$Me-Leu; and $R_6$ is Gly-$NH_2$ or $NHCH_2CH_3$.

By dehydro Pro is meant 3,4 dehydroproline, $C_5H_7O_2N$, and when X is an acyl radical, it is attached to the nitrogen. By Thz is meant meta-thiazolidine-2-carboxylic acid, $C_4H_7O_2NS$, which can be prepared by the treatment of cysteine hydrochloride with formaldehyde, and for example, Ac-Thz may be prepared by the reaction of Thz with acetic anhydride.

The peptides of the present invention can be synthesized by a solid phase technique using a chloromethylated resin, a methylbenzhydrylamine resin (MBHA) or a benzhydrylamine (BHA) resin. The synthesis is conducted in a manner to stepwise add the amino acids in the chain in the manner set forth in detail in the U.S. Patent No. 4,211,693. Side-chain protecting groups, as are well known in the art, are preferably added to Ser, Tyr, Arg and His before these amino acids are coupled to the chain being built upon the resin.

Such a method provides the fully protected intermediate peptidoresin. The intermediates of the invention may be represented as:

$$X^1\text{-}R_1\text{-}R_2\text{-}R_3\text{-Ser}(X^2)\text{-Tyr}(X^3)\text{-}R_4\text{-}R_5\text{-Arg}(X^4)\text{-Pro-}X^5$$

wherein: $X^1$ is an $\alpha$-amino protecting group of the type known to be useful in the art in the stepwise synthesis of polypeptides and when X in the desired peptide composition is a particular acyl group, that group may be used as the protecting group. Among the classes of $\alpha$-amino protecting groups covered by $X^1$ are (1) acyl-type protecting groups, such as formyl (For), trifluoroacetyl, phthalyl, p-toluenesulfonyl (Tos), benzoyl (Bz), benzensulfonyl, o-nitrophenylsulfenyl (Nps), tritylsulfenyl, o-nitrophenoxyacetyl, acrylyl (Acr), chloroacetyl, acetyl (Ac) and $\gamma$-chlorobutyryl; (2) aromatic urethan-type protecting groups, e.g., benzyloxycarbonyl (Z) and substituted benzyloxycarbonyl, such as p-chlorobenzyloxycarbonyl, p-nitrobenzyloxycarbonyl, p-bromobenzyloxycarbonyl and p-methoxybenzyloxycarbonyl; (3) aliphatic urethan protecting groups, such as tertbutyloxycarbonyl (Boc), diisopropylmethoxycarbonyl, isopropyloxycarbonyl, ethoxycarbonyl and allyloxycarbonyl; (4) cycloalkyl urethan-type protecting groups, such as cyclopentyloxycarbonyl, adamantyloxycarbonyl and cyclohexyloxycarbonyl; (5) thiourethan-type protecting groups, such as phenylthiocarbonyl; (6) alkyl-type protecting

groups, such as allyl (Aly), triphenylmethyl(trityl) and benzyl (Bzl); (7) trialkylsilane groups, such as trimethylsilane. The preferred $\alpha$-amino protecting group is Boc when X is hydrogen.

$X^2$ is a protecting group for the alcoholic hydroxyl group of Ser and is selected from the group consisting of acetyl, benzoyl, tetrahydropyranyl, tert-butyl, trityl, benzyl and 2,6-dichlorobenzyl. Benzyl is preferred.

$X^3$ is a protecting group for the phenolic hydroxyl group of Tyr selected from the group consisting of tetrahydropyranyl, tert-butyl, trityl, benzyl, benzyloxycarbonyl, 4-bromobenzyloxycarbonyl and 2,6-dichlorobenzyl.

$X^4$ is a protecting group for the nitrogen atoms of Arg and is selected from the group consisting of nitro, Tos, benzyloxycarbonyl, adamantyloxycarbonyl, and Boc; alternatively $X^4$ may be hydrogen, which means there are no protecting groups on the side chain nitrogen atoms of arginine.

$X^5$ is selected from Gly-O-$CH_2$-[resin support]; O-$CH_2$-[resin support] and Gly-NH-[resin support].

The criterion for selecting side chain protecting groups for $X^2$—$X^4$ is that the protecting group must be stable to the reagent under the reaction conditions selected for removing the $\alpha$-amino protecting group at each step of the synthesis. The protecting group must not be split off under coupling conditions, and the protecting group must be removable upon completion of the synthesis of the desired amino acid sequence under reaction conditions that will not alter the peptide chain.

When the $X^5$ group is Gly-O-$CH_2$-[resin support], the ester moiety of one of the many functional groups of the polystyrene resin support is being represented. When the $X^5$ group is Gly-NH-[resin support], an amide bond connects Gly to BHA resin or to a MBHA resin.

When X is acetyl, formyl, acrylyl, vinylacetyl, benzoyl or some other acyl group having 7 carbon atoms or less, it may be employed as the $X^1$ protecting group for the $\alpha$-amino group of $R_1$ in which case it can be added before coupling of the last amino acid to the peptide chain. Alternatively, a reaction may be carried out with the peptide on the resin, e.g. reacting with acetic acid in the presence of dicyclohexyl carbodiimide (DCC) or preferably with acetic anhydride.

The fully protected peptide can be cleaved from the chloromethylated resin support by ammonolysis, as is well known in the art, to yield the fully protected amide intermediate. Deprotection of the peptide as well as cleavage of the peptide from the benzhydrylamine resin can take place at 0°C with hydrofluoric acid (HF). Anisole can be added to the peptide prior to treatment with HF. After the removal of HF, under vacuum, conveniently the cleaved, deprotected peptide is treated with ether, decanted, taken in dilute acetic acid and lyophilized.

Purification of the peptide can be effected by ion exchange chromotography on a CMC column, followed by partition chromotography using the elution system: n-butanol; 0.1N acetic acid (1:1 volume ratio) on a column packed with Sephadex® G-25, or by using HPLC, as known in the arts. The peptides of the invention are effective at levels of less than 200 micrograms per kilogram of body weight, when administered at about noon on the day of proestrous, to prevent ovulation in female rats. For prolonged suppression of ovulation, it may be necessary to use dosage levels in the range of from about 0.1 to about 5 milligrams per kilogram of body weight. These antagonists are also effective as contraceptives when administered to male mammals on a regular basis.

Since these compounds will reduce testosterone levels (an undesired consequence in the normal, sexually active male), it may be reasonable to administer replacement dosages of testosterone along with the LRF antagonist.

The following Examples are intended further to illustrate the invention by way of example only. In the Examples, Ac=acetyl, Bz=benzyl, Acr=acrylyl, For=formyl.

Example I

The following peptides having the formula

X-dehydro Pro-$R_2$-D-Trp-Ser-Tyr-D-Trp-$R_5$-Arg-Pro-Gly-$NH_2$

are prepared by the solid phase procedure referred to above.

TABLE I

| Peptide | X | $R_2$ | $R_5$ |
|---|---|---|---|
| 1 | Ac | 3,4 $Cl_2$-D-Phe | Leu |
| 2 | " | 4 $CF_3$-D-Phe | " |
| 3 | " | 4F-D-Phe | " |
| 4 | " | 4 AcNH-D-Phe | " |
| 5 | " | 4 $NO_2$-D-Phe | " |
| 6 | " | 4 Br-D-Phe | " |
| 7 | " | 4 $CH_3S$-D-Phe | " |
| 8 | " | 4 $OCH_3$-D-Phe | " |
| 9 | " | 4 $CH_3$-D-Phe | " |
| 10 | " | 2,4 $Cl_2$-D-Phe | " |
| 11 | Acr | 3,4 $Cl_2$-D-Phe | " |
| 12 | Ac | 4 $OCH_3$-D-Phe | $N^\alpha$MeLeu |
| 13 | " | 4 $CH_3$-D-Phe | " |
| 14 | " | 3,4 $Cl_2$-D-Phe | " |

For purposes of an example, a representative solid phase synthesis of Peptide No. 1 above, which is referred to as [Ac-dehydro $Pro^1$, 3,4 $Cl_2$-D-$Phe^2$,D-$Trp^{3,6}$]-LRF is set forth hereinafter. This peptide has the following formula:

Ac-dehydro Pro-3,4 $Cl_2$-D-Phe-D-Trp-Ser-Tyr-D-Trp-Leu-Arg-Pro-Gly-$NH_2$

A BHA resin is used, and Boc-protected Gly is coupled to the resin over a 2-hour period in $CH_2Cl_2$ using a 3-fold excess of Boc derivative and DCC as an activating reagent. The glycine residue attaches to the BHA residue by an amide bond.

Following the coupling of each amino acid residue, washing, deblocking and coupling of the next amino acid residue is carried out in accordance with the following schedule using an automated machine and beginning with about 5 grams of resin:

| Step | Reagents and operations | Mix times min. |
|---|---|---|
| 1 | $CH_2Cl_2$ wash-80 ml. (2 times) | 3 |
| 2 | Methanol (MeOH) wash-30 ml. (2 times) | 3 |
| 3 | $CH_2Cl_2$ wash-80 ml. (3 times) | 3 |
| 4 | 50 percent TFA plus 5 percent 1,2-ethanedithiol in $CH_2Cl_2$-70 ml. (2 times) | 10 |
| 5 | $CH_2Cl_2$ wash-80 ml. (2 times) | 3 |
| 6 | TEA 12.5 percent in $CH_2Cl_2$-70 ml. (2 times) | 5 |
| 7 | MeOH wash-40 ml. (2 times) | 2 |
| 8 | $CH_2Cl_2$ wash-80 ml. (3 times) | 3 |
| 9 | Boc-amino acid (10 mmoles) in 30 ml. of either DMF or $CH_2Cl_2$, depending upon the solubility of the particular protected amino acid, (1 time) plus DCC (10 mmoles) in $CH_2Cl_2$ | 30—300 |
| 10 | MeOH wash-40 ml. (2 times) | 3 |
| 11 | TEA 12.5 percent in $CH_2Cl_2$-70 ml. (1 time) | 3 |
| 12 | MeOH wash-30 ml. (2 times) | 3 |
| 13 | $CH_2Cl_2$ wash-80 ml. (2 times) | 3 |

After step 13, an aliquot is taken for a ninhydrin test: if the test is negative, go back to step 1 for coupling of the next amino acid; if the test is positive or slightly positive, go back and repeat steps 9 through 13.

The above schedule is used for coupling of each of the amino acids of the peptide of the invention after the first amino acid has been attached. $N^\alpha$Boc protection is used for each of the remaining amino acids throughout the synthesis. The side chain of Arg is protected with Tos. OBzl is used as a side chain protecting group for the hydroxyl group of Ser, and 2—6 dichlorobenzyl is used as the side chain protecting group for the hydroxyl group of Tyr. N-acetyl-dehydro Pro is introduced as the final amino acid. Boc-Arg(Tos) and Boc-D-Trp, which have low solubility in $CH_2Cl_2$, are coupled using DMF $CH_2Cl_2$ · mixtures.

The cleavage of the peptide from the resin and complete deprotection of the side chains takes place very readily at 0°C. with HF. Anisole is added as a scavenger prior to HF treatment. After the removal of HF under vacuum, the resin is extracted with 50% acetic acid, and the washings are lyophilized to provide a crude peptide powder.

Purification of the peptide is then effected by ion exchange chromatography on CMC (Whatman CM 32, using a gradient of 0.05 to 0.3M $NH_4OAc$ in 50/50 methanol/water) followed by partition

chromatography in a gel filtration column using the elution system: n-Butanol; 0.1N Acetic acid (1:1—volume ratio).

The peptides set forth in the foregoing table are assayed *in vitro* and *in vivo.* The *in vitro* test is made using dissociated rat pituitary cells maintained in culture for 4 days prior to the assay. The levels of LH mediated in response to the application of peptides is assayed by specific radioimmunoassay for rat LH. Control dishes of cells only receive a measure which is 3 nanomolar in LRF: experimental dishes receive a measure 3 nanomolar in LRF plus a measure having a concentration of test peptide ranging from 0.01 to 3 nanomolar. The amount of LH secreted in the samples treated only with LRF is compared with that secreted by the samples treated with the peptide plus LRF. Results are calculated and expressed in Table IA (In Vitro column) as the molar concentration ratio of test peptide to LRF (antagonist/LRF) required to reduce the amount of LH released by 3 nanomolar LRF to 50 percent of the control value ($ICR_{50}$).

The peptides described hereinabove are used to determine effectiveness to prevent ovulation in female rats. In this test, a specified number of mature female Sprague-Dawley rats, each having a body weight from 225 to 250 grams, are injected with either 5 or 10 micrograms of peptide in corn oil at about noon on the day of proestrous. Proestrous is the afternoon before estrous (ovulation). A separate female rat group is used as a control to which the peptide is not administered. Each of the control rat females has ovulation at estrous. As indicated in the In Vivo column, the peptides are significantly effective to prevent ovulation of female rats at a very low dosage, and all of the peptide compositions are considered to be totally effective at a dose of one milligram.

TABLE IA

| Peptide | In Vitro $ICR_{50}$ | In Vivo (10 μg) rats ovulating | In Vivo (5 μg) rats ovulating |
|---|---|---|---|
| 1 | 0.039 | 0/10 | 4/7 |
| 2 | 0.070 | | 7/9 |
| 3 | 0.021 | 0/10 | 0/10 |
| 4 | | 9/10 | |
| 5 | 0.011 | 0/10 | 2/10 |
| 6 | 0.010 | | 5/7 |
| 7 | 0.030 | 1/10 | 7/10 |
| 8 | 0.125 | 3/10 | |
| 9 | 0.044 | 1/7 | 9/9 |
| 10 | | 5/10 | 4/7 |
| 11 | 0.048 | 1/10 | 4/10 |
| 12 | 0.29 | 5/10 | |
| 13 | 0.11 | 3/4 | |
| 14 | 0.05 | 1/3 | 8/10 |

Example II

The following peptides having the formula

$$X\text{-}R_1\text{-pCl-D-Phe-D-Trp-Ser-Tyr-}R_4\text{-}R_5\text{-Arg-Pro-}R_6$$

are prepared by the solid phase procedure as generally described in Example I except for No. 20 which is prepared on a chloromethylated resin.

TABLE II

| Peptide | X | $R_1$ | $R_4$ | $R_5$ | $R_6$ |
|---|---|---|---|---|---|
| 15 | Ac | dehydro Pro | D-Trp | Leu | $Gly\text{-}NH_2$ |
| 16 | " | " | " | $N^{\alpha}MeLeu$ | " |
| 17 | " | dehydro D-Pro | " | Leu | " |
| 18 | " | " | " | $N^{\alpha}MeLeu$ | " |
| 19 | Acr | dehydro Pro | " | " | " |
| 20 | Ac | dehydro D-Pro | " | Leu | $NHCH_2CH_3$ |
| 21 | Ac | Thz | D-Trp | Leu | $Gly\text{-}NH_2$ |
| 22 | " | " | " | $N^{\alpha}MeLeu$ | " |
| 23 | " | D-Thz | " | " | " |
| 24 | " | " | " | Leu | " |
| 25 | " | dehydro Pro | (imBzl)D-His | " | " |
| 26 | " | dehydro D-Pro | " | " | " |
| 27 | " | Thz | " | " | " |
| 28 | " | D-Thz | " | " | " |

The peptides set forth in the foregoing table are assayed *in vitro* and *in vivo*. The *in vitro* test is made using dissociated rat pituitary cells maintained in culture for 4 days prior to the assay. The levels of LH mediated in response to the application of peptides is assayed by specific radioimmunoassay for rat LH. Control dishes of cells only receive a measure which is 3 nanomolar in LRF: experimental dishes receive a measure 3 nanomolar in LRF plus a measure having a concentration of test peptide ranging from 1 to 100 nanomolar. The amount of LH secreted in the samples treated only with LRF is compared with that secreted by the samples treated with the peptide plus LRF. Results are calculated and expressed in Table IIA (In Vitro column) as the molar concentration ratio of test peptide to LRF (antagonist/LRF) required to reduce the amount of LH released by 3 nanomolar LRF to 50 percent of the control value ($ICR_{50}$).

Several of the peptides described hereinabove are used to determine effectiveness to prevent ovulation in female rats. In this test, four, seven, nine or ten mature female Sprague-Dawley rats, each having a body weight from 225 to 250 grams, are injected with 0.02 milligram of peptide (unless otherwise indicated) in corn oil at about noon on the day of proestrous. Proestrous is the afternoon before estrous (ovulation). A separate ten female rat group is used as a control to which the peptide is not administered. Each of the ten control rat females has ovulation at estrous. As indicated in the In Vivo column, the peptides are significantly effective to prevent ovulation of female rats at a very low dosage, and all of the peptide compositions are considered to be totally effective at a dose of one milligram.

TABLE IIA

| Peptide | In Vitro $ICR_{50}$ | In Vivo rats ovulating |
|---|---|---|
| 15 | 0.043 | 0/10* |
| 16 | 0.058 | 0/7 |
| 17 | 0.19 | 3/8 |
| 18 | 0.27 | 5/10** |
| 19 | 0.03 | 0/9 |
| 20 | 0.2 | |
| 21 | 0.14 | 5/8 |
| 22 | 0.13 | 9/10** |
| 23 | 0.1 | |
| 24 | 0.12 | |
| 25 | 0.042 | 4/10 |
| 26 | 0.2 | 10/10 |
| 27 | 0.15 | 7/10** |
| 28 | 0.1 | 7/10** |

**0.025 mg.
*0.010 mg.

Example III

The following peptides having the formula

$$X\text{-}R_1\text{-}R_2\text{-}R_3\text{-Ser-Tyr-D-Trp-}R_5\text{-Arg-Pro-}R_6$$

are prepared by a solid phase procedure generally as described in Example I, except for No. 37 where a chloromethylated resin is used.

TABLE III

| Peptide | X | $R_1$ | $R_2$ | $R_3$ | $R_5$ | $R_6$ |
|---|---|---|---|---|---|---|
| 29 | H | dehydro-D,L-Pro | D-Phe | D-Trp | $N^\alpha$MeLeu | Gly-$NH_2$ |
| 30 | H | " | " | " | Leu | " |
| 31 | Ac | dehydro-D,L-Pro | " | " | " | " |
| 32 | H | " | " | " | $N^\alpha$MeLeu | " |
| 33 | Ac | dehydro-Pro | " | " | Leu | " |
| 34 | Ac | dehydro-D-Pro | " | " | " | " |
| 35 | H | dehydro-Pro | " | " | " | " |
| 36 | H | dehydro-D-Pro | " | " | " | " |
| 37 | H | dehydro-Pro | " | " | " | $NHCH_2CH_3$ |
| 38 | Bz | dehydro-Pro | D-Trp | " | " | Gly-$NH_2$ |
| 39 | For | dehydro-Pro | " | D-Phe | " | " |
| 40 | For | dehydro-Pro | D-His | Trp | " | " |
| 41 | Acr | dehydro-Pro | " | " | " | " |
| 42 | Bz | dehydro-Pro | Trp | D-His | " | " |
| 43 | Acr | dehydro-Pro | " | D-Trp | " | " |
| 44 | For | dehydro-Pro | " | D-His | " | " |
| 45 | Bz | dehydro-Pro | D-His | " | " | " |

The peptides set forth in the foregoing table are assayed *in vitro* and *in vivo*. The *in vitro* assay is made using four day old primary culture of dispersed rat pituitary cells. The levels of LH mediated in response to the application of peptides is assayed by specific radioimmunoassay for rat LH. Control dishes of cells only receive a measure which is 3 nanomolar in LRF: experimental dishes receive a measure 3 nanamoles of LRF and a concentration of test peptide ranging from 1 to 100 nanomolar. The amount of LH secreted in the samples treated only with LRF is compared with that secreted by the samples treated with the peptide plus LRF. Results are calculated and expressed in Table IIIA (In Vitro column) as the molar concentration ratio of test peptide required to reduce the amount of LH released by the 3 nanomolar LRF to 50 percent of the control value ($ICR_{50}$).

Several of the peptides described hereinabove are used to determine effectiveness to prevent ovulation in female rats. In this test, a number of mature female Sprague-Dawley rats, each having a body weight from 225 to 250 grams, are injected with 0.02 milligram of peptide in corn oil at about noon on the day of proestrous. Proestrous in the afternoon before estrous (ovulation). A separate female rat group is used as a control to which the peptide is not administered. Each of the control rat females has ovulation at estrous. As indicated in the In Vivo column, the peptides are significantly effective to prevent ovulation of female rats at a very low dosage, and all of the peptide compositions are considered to be totally effective at a dose of one milligram.

TABLE IIIA

| Peptide | In Vivo $icr_{50}$ | In Vitro rats ovulating |
|---|---|---|
| 29 | 0.5:1 | 4/10 |
| 30 | 0.8:1 | 7/10 |
| 31 | 0.3:1 | 1/10 |
| 32 | 0.4:1 | 2/10 |
| 33 | 0.6:1 | 0/4* |

*dose of 0.025 mg.

The peptides of the invention can be administered to mammals intravenously, subcutaneously, intramuscularly, orally, intranasally or intravaginally to achieve fertility inhibition and/or control. Effective dosages will vary with the form of administration and the particular species of mammal being treated. An example of one typical dosage form is a physiological saline solution containing the peptide which solution is administered to provide a dose in the range of about 0.1 to 5 mg/kg of body weight. Oral administration of the peptide may be given in either solid form or liquid form.

Although the invention has been described with regard to its preferred embodiments, it should be understood that changes and modifications as would be obvious to one having the ordinary skill in this art may be made without departing from the scope of the invention which is set forth in the claims which are appended hereto. For example, other substitutions known in the art which do not significantly detract from the effectiveness of the peptides may be employed in the peptides of the invention.

**Claims for the Contracting States: BE CH DE FR GB IT LI LU NL SE**

1. A peptide (or a nontoxic salt thereof), said peptide having the formula:

$$X\text{-}R_1\text{-}R_2\text{-}R_3\text{-Ser-Tyr-}R_4\text{-}R_5\text{-Arg-Pro-}R_6$$

wherein X is hydrogen or an acyl group having 7 or less carbon atoms; $R_1$ is dehydro Pro, dehydro D-Pro, Thz or D-Thz; $R_2$ is D-Phe, D-His, D-Trp, Trp, Cl-D-Phe, dichloro-D-Phe, $CF_3$-D-Phe, F-D-Phe, difluoro-D-Phe, AcNH-D-Phe, $NO_2$-D-Phe, dinitro-D-Phe, Br-D-Phe, dibromo-D-Phe, $CH_3S$-D-Phe, $OCH_3$-D-Phe or $CH_3$-D-Phe; $R_3$ is D-Trp, Trp, D-Phe or D-His; $R_4$ is Gly or a D-isomer amino acid; $R_5$ is Leu or $N^\alpha$me-Leu; and $R_6$ is Gly-$NH_2$ or $NHCH_2CH_3$.

2. A peptide in accordance with Claim 1 wherein $R_2$ is dichloro-D-Phe, 4 $CF_3$-D-Phe, 4 F-D-Phe, difluoro-D-Phe, 4 AcNH-D-Phe, 4 $NO_2$-D-Phe, dinitro-D-Phe, 4 Br-D-Phe, dibromo-D-Phe, 4 $CH_3S$-D-Phe, 4 $OCH_3$-D-Phe or 4 $CH_3$-D-Phe.

3. A peptide in accordance with either Claim 1 or 2 wherein $R_1$ is dehydro-Pro.

4. A peptide in accordance with any of Claims 1, 2 and 3 wherein X is acrylyl.

5. A peptide in accordance with any of Claims 1, 2 and 3 wherein X is acetyl.

6. A peptide in accordance with any of Claims 1 to 5 wherein $R_3$ is D-Trp.

7. A peptide in accordance with any of Claims 1 to 6 wherein $R_4$ is D-Trp.

8. A peptide in accordance with any of Claims 1 to 6 wherein $R_4$ is a lipophilic, aromatic D-isomer amino acid.

9. A peptide in accordance with any of Claims 1 to 8 wherein $R_5$ is Leu.

10. A peptide in accordance with any of Claims 1 to 9 wherein $R_6$ is Gly-$NH_2$.

11. A peptide as listed (Peptides 1—14) in the Table below and defined by the following general formula:—

$$X\text{-dehydro Pro-}R_2\text{-D-Trp-Ser-Tyr-D-Trp-}R_5\text{-Arg-Pro-Gly-}NH_2$$

| Peptide | X | $R_2$ | $R_5$ |
|---|---|---|---|
| 1 | Ac | 3,4 $Cl_2$-D-Phe | Leu |
| 2 | " | 4 $CF_3$-D-Phe | " |
| 3 | " | 4F-D-Phe | " |
| 4 | " | 4 AcNH-D-Phe | " |
| 5 | " | 4 $NO_2$-D-Phe | " |
| 6 | " | 4 Br-D-Phe | " |
| 7 | " | 4 $CH_3S$-D-Phe | " |
| 8 | " | 4 $OCH_3$-D-Phe | " |
| 9 | " | 4 $CH_3$-D-Phe | " |
| 10 | " | 2,4 $Cl_2$-D-Phe | " |
| 11 | Acr | 3,4 $Cl_2$-D-Phe | " |
| 12 | Ac | 4 $OCH_3$-D-Phe | $N^\alpha$MeLeu |
| 13 | " | 4 $CH_3$-D-Phe | " |
| 14 | " | 3,4 $Cl_2$-D-Phe | " |

12. A peptide as listed (Peptides 15—28) in the Table below and defined by the following general formula:—

$$X\text{-}R_1\text{-p-Cl-D-Phe-D-Trp-Ser-Tyr-}R_4\text{-}R_5\text{-Arg-Pro-}R_6$$

| Peptide | X | $R_1$ | $R_4$ | $R_5$ | $R_6$ |
|---|---|---|---|---|---|
| 15 | Ac | dehydro Pro | D-Trp | Leu | Gly-$NH_2$ |
| 16 | " | " | " | $N^\alpha$MeLeu | " |
| 17 | " | dehydro D-Pro | " | Leu | " |
| 18 | " | " | " | $N^\alpha$MeLeu | " |
| 19 | Acr | dehydro Pro | " | " | " |
| 20 | Ac | dehydro D-Pro | " | Leu | $NHCH_2CH_3$ |
| 21 | Ac | Thz | D-Trp | Leu | Gly-$NH_2$ |
| 22 | " | " | " | $N^\alpha$MeLeu | " |
| 23 | " | D-Thz | " | " | " |
| 24 | " | " | " | Leu | " |
| 25 | " | dehydro Pro | (imBzl)D-His | " | " |
| 26 | " | dehydro D-Pro | " | " | " |
| 27 | " | Thz | " | " | " |
| 28 | " | D-Thz | " | " | " |

13. A peptide as listed (Peptides 29—45) in the Table below and defined by the following general formula:—

$$X\text{-}R_1\text{-}R_2\text{-}R_3\text{-Ser-Tyr-D-Trp-}R_5\text{-Arg-Pro-}R_6$$

| Peptide | X | $R_1$ | $R_2$ | $R_3$ | $R_5$ | $R_6$ |
|---|---|---|---|---|---|---|
| 29 | H | dehydro-D,L-Pro | D-Phe | D-Trp | $N^{\alpha}$MeLeu | Gly-$NH_2$ |
| 30 | H | " | " | " | Leu | " |
| 31 | Ac | dehydro-D,L-Pro | " | " | " | " |
| 32 | H | " | " | " | $N^{\alpha}$MeLeu | " |
| 33 | Ac | dehydro-Pro | " | " | Leu | " |
| 34 | Ac | dehydro-D-Pro | " | " | " | " |
| 35 | H | dehydro-Pro | " | " | " | " |
| 36 | H | dehydro-D-Pro | " | " | " | " |
| 37 | H | dehydro-Pro | " | " | " | $NHCH_2CH_3$ |
| 38 | Bz | dehydro-Pro | D-Trp | " | " | Gly-$NH_2$ |
| 39 | For | dehydro-Pro | " | D-Phe | " | " |
| 40 | For | dehydro-Pro | D-His | Trp | " | " |
| 41 | Acr | dehydro-Pro | " | " | " | " |
| 42 | Bz | dehydro-Pro | Trp | D-His | " | " |
| 43 | Acr | dehydro-Pro | " | D-Trp | " | " |
| 44 | For | dehydro-Pro | " | D-His | " | " |
| 45 | Bz | dehydro-Pro | D-His | " | " | " |

14. A method for the manufacture of a compound having the formula:

$$X\text{-}R_1\text{-}R_2\text{-}R_3\text{-Ser-Tyr-}R_4\text{-}R_5\text{-Arg-Pro-}R_6$$

wherein X is hydrogen or an acyl group having 7 or less carbon atoms; $R_1$ is dehydro Pro, dehydro D-Pro, Thz or D-Thz; $R_2$ is D-Phe, D-His, D-Trp, Trp, Cl-D-Phe, dichloro-D-Phe, $CF_3$-D-Phe, F-D-Phe, difluoro-D-Phe, AcNH-D-Phe, $NO_2$-D-Phe, dinitro-D-Phe, Br-D-Phe, dibromo-D-Phe, $CH_3S$-D-Phe, $OCH_3$-D-Phe or $CH_3$-D-Phe; $R_3$ is D-Trp, Trp, D-Phe or D-His; $R_4$ is Gly or a D-isomer amino acid; $R_5$ is Leu or $N^{\alpha}$Me-Leu; and $R_6$ is Gly-$NH_2$ or $NHCH_2CH_3$, the process comprising;

(a) forming an intermediate compound having the formula:

$$X^1\text{-}R_1\text{-}R_2\text{-}R_3\text{-Ser}(X^2)\text{-Tyr-}(X^3)\text{-}R_4\text{-}R_5\text{-Arg}(X^4)\text{-Pro-}X^5$$

wherein $X^1$ is an $\alpha$-amino protecting group; $X^2$ is a protecting group for the alcoholic hydroxyl group of Ser; $X^3$ is a protecting group for the phenolic hydroxyl group of Tyr; $X^4$ is a protecting group for the nitrogen atoms of Arg; and $X^5$ is selected from Gly-O-$CH_2$ (resin support), O-$CH_2$-(resin support), Gly-NH-(resin support), Gly-$NH_2$ and $NHCH_2CH_3$;

(b) splitting off one or more of the groups $X^1$ to $X^5$ and, if desired, converting a resulting peptide into a nontoxic salt thereof.

15. A pharmaceutical composition comprising a peptide as claimed in any one of Claims 1 to 13 together with a carrier or diluent rendering the composition administrable intravenously, subcutaneously, intramuscularly, orally, intranasally or intravaginally.

**Claims for the Contracting State: AT**

1. A method for the manufacture of a compound having the formula:

$$X\text{-}R_1\text{-}R_2\text{-}R_3\text{-Ser-Tyr-}R_4\text{-}R_5\text{-Arg-Pro-}R_6$$

wherein X is hydrogen or an acyl group having 7 or less carbon atoms; $R_1$ is dehydro Pro, dehydro D-Pro, Thz or D-Thz; $R_2$ is D-Phe, D-His, D-Trp, Trp, Cl-D-Phe, dichloro-D-Phe, $CF_3$-D-Phe, F-D-Phe, difluoro-D-Phe, AcNH-D-Phe, $NO_2$-D-Phe, dinitro-D-Phe, Br-D-Phe, dibromo-D-Phe, $CH_3S$-D-Phe, $OCH_3$-D-Phe or $CH_3$-D-Phe; $R_3$ is D-Trp, Trp, D-Phe or D-His; $R_4$ is Gly or a D-isomer amino acid; $R_5$ is Leu or $N^{\alpha}$Me-Leu; and $R_6$ is Gly-$NH_2$ or $NHCH_2CH_3$.

comprising (a) forming an intermediate compound having the formula:

$$X^1\text{-}R_1\text{-}R_2\text{-}R_3\text{-Ser}(X^2)\text{-Tyr-}(X^3)\text{-}R_4\text{-}R_5\text{-Arg}(X^4)\text{-Pro-}X^5$$

wherein $X^1$ is an $\alpha$-amino protecting group; $X^2$ is a protecting group for the alcoholic hydroxyl group of Ser; $X^3$ is a protecting group for the phenolic hydroxyl group of Tyr; $X^4$ is a protecting group for the

nitrogen atoms of Arg; and $X^5$ is selected from Gly-O-$CH_2$[resin support], O-$CH_2$-[resin support], Gly-NH-[resin support], Gly-$NH_2$ and $NHCH_2CH_3$;

(b) splitting off one or more of the groups $X^1$ to $X^5$ and, if desired, converting a resulting peptide into a nontoxic salt thereof.

2. A method in accordance with Claim 1 wherein $R_2$ is dichloro-D-Phe, 4 $CF_3$-D-Phe, 4 F-D-Phe, difluoro-D-Phe, 4 AcNH-D-Phe, 4 $NO_2$-D-Phe, dinitro-D-Phe, 4 Br-D-Phe, dibromo-D-Phe, 4 $CH_3S$-D-Phe, 4 $OCH_3$-D-Phe or 4 $CH_3$-D-Phe.

3. A method in accordance with either Claim 1 or 2 wherein $R_1$ is dehydro-Pro.

4. A method in accordance with any of Claims 1, 2 and 3 wherein X is acrylyl.

5. A method in accordance with any of Claims 1, 2 and 3 wherein X is acetyl.

6. A method in accordance with any of Claims 1 to 5 wherein $R_3$ is D-Trp.

7. A method in accordance with any of Claims 1 to 6 wherein $R_4$ is D-Trp.

8. A method in accordance with any of Claims 1 to 6 wherein $R_4$ is a lipophilic, aromatic D-isomer amino acid.

9. A method in accordance with any of Claims 1 to 8 wherein $R_5$ is Leu.

10. A method in accordance with any of Claims 1 to 9 wherein $R_6$ is Gly-$NH_2$.

**Patentansprüche: für die Vertragsstaaten: BE CH DE FR GB IT LI LU NL SE**

1. Ein Peptid (oder ein nicht-toxisches Salz hievon) der Formel:

X-$R_1$-$R_2$-$R_3$-Ser-Tyr-$R_4$-$R_5$-Arg-Pro-$R_6$

worin X für Wasserstoff oder eine Acylgruppe mit 7 oder weniger Kohlenstoffatomen, $R_1$ für Dehydro-Pro, Dehydro-D-Pro, Thz oder D-Thz, $R_2$ für D-Phe, D-His, D-Trp, Trp, Cl-D-Phe, Dichlor-D-Phe, $CF_3$-D-Phe, F-D-Phe, Difluor-D-Phe, AcNH-D-Phe, $NO_2$-D-Phe, Dinitro-D-Phe, Br-D-Phe, Dibrom-D-Phe, $CH_3S$-D-Phe, $OCH_3$-D-Phe oder $CH_3$-D-Phe, $R_3$ für D-Trp, Trp, D-Phe oder D-His, $R_4$ für Gly oder eine D-isomere Aminosäure, $R_5$ für Leu oder $N^\alpha$Me-Leu und $R_6$ für Gly-$NH_2$ oder $NHCH_2CH_3$ steht.

2. Ein Peptid nach Anspruch 1, worin $R_2$ für Dichlor-D-Phe, 4-$CF_3$-D-Phe, 4-F-D-Phe, Difluor-D-Phe, 4-AcNH-D-Phe, 4-$NO_2$-D-Phe, Dinitro-D-Phe, 4-Br-D-Phe, Dibrom-D-Phe, 4-$CH_3S$-D-Phe, 4-$OCH_3$-D-Phe oder 4-$CH_3$-D-Phe steht.

3. Ein Peptid nach Anspruch 1 oder 2, worin $R_1$ für Dehydro-Pro steht.

4. Ein Peptid nach irgendeinem der Ansprüche 1, 2 und 3, worin X für Acrylyl steht.

5. Ein Peptid nach irgendeinem der Ansprüche 1, 2 und 3, worin X für Acetyl steht.

6. Ein Peptid nach irgendeinem der Ansprüche 1 bis 5, worin $R_3$ für D-Trp steht.

7. Ein Peptid nach irgendeinem der Ansprüche 1 bis 6, worin $R_4$ für D-Trp steht.

8. Ein Peptid nach irgendeinem der Ansprüche 1 bis 6, worin $R_4$ für eine lipophile, aromatische D-isomere Aminosäure steht.

9. Ein Peptid nach irgendeinem der Ansprüche 1 bis 8, worin $R_5$ für Leu steht.

10. Ein Peptid nach irgendeinem der Ansprüche 1 bis 9, worin $R_6$ für Gly-$NH_2$ steht.

11. Ein in der unterstehenden Tabelle angegebenes und durch die folgende allgemeine Formel

X-dehydro-Pro-$R_2$-D-Trp-Ser-Tyr-D-Trp-$R_5$-Arg-Pro-Gly-$NH_2$

definiertes Peptid (Peptide 1—14):

| Peptid | X | $R_2$ | $R_5$ |
|---|---|---|---|
| 1 | Ac | 3,4-$Cl_2$-D-Phe | Leu |
| 2 | " | 4-$CF_3$-D-Phe | " |
| 3 | " | 4F-D-Phe | " |
| 4 | " | 4-AcNH-D-Phe | " |
| 5 | " | 4-$NO_2$-D-Phe | " |
| 6 | " | 4-Br-D-Phe | " |
| 7 | " | 4-$CH_3S$-D-Phe | " |
| 8 | " | 4-$OCH_3$-D-Phe | " |
| 9 | " | 4-$CH_3$-D-Phe | " |
| 10 | " | 2,4-$Cl_2$-D-Phe | " |
| 11 | Acr | 3,4-$Cl_2$-D-Phe | " |
| 12 | Ac | 4-$OCH_3$-D-Phe | $N^\alpha$MeLeu |
| 13 | " | 4-$CH_3$-D-Phe | " |
| 14 | " | 3,4-$Cl_2$-D-Phe | " |

12 Ein in der unten stehenden Tabelle angegebenes und durch die folgende allgemeine Formel

$$X\text{-}R_1\text{-p-Cl-D-Phe-D-Trp-Ser-Tyr-}R_4\text{-}R_5\text{-Arg-Pro-}R_6$$

definiertes Peptid (Peptide 15—28):

| Peptid | X | $R_1$ | $R_4$ | $R_5$ | $R_6$ |
|---|---|---|---|---|---|
| 15 | Ac | Dehydro-Pro | D-Trp | Leu | $Gly\text{-}NH_2$ |
| 16 | " | " | " | $N^\alpha$MeLeu | " |
| 17 | " | Dehydro-D-Pro | " | Leu | " |
| 18 | " | " | " | $N^\alpha$MeLeu | " |
| 19 | Acr | Dehydro-Pro | " | " | " |
| 20 | Ac | Dehydro-D-Pro | " | Leu | $NHCH_2CH_3$ |
| 21 | Ac | Thz | D-Trp | Leu | $Gly\text{-}NH_2$ |
| 22 | " | " | " | $N^\alpha$MeLeu | " |
| 23 | " | D-Thz | " | " | " |
| 24 | " | " | " | Leu | " |
| 25 | " | Dehydro-Pro | (imBzl)D-His | " | " |
| 26 | " | Dehydro-D-Pro | " | " | " |
| 27 | " | Thz | " | " | " |
| 28 | " | D-Thz | " | " | " |

13. Ein in der unterstehenden Tabelle angegebenes und durch die folgende allgemeine Formel

$$X\text{-}R_1\text{-}R_2\text{-}R_3\text{-Ser-Tyr-D-Trp-}R_5\text{-Arg-Pro-}R_6$$

definiertes Peptid (Peptide 29—45):

| Peptid | X | $R_1$ | $R_2$ | $R_3$ | $R_5$ | $R_6$ |
|---|---|---|---|---|---|---|
| 29 | H | Dehydro-D,L-Pro | D-Phe | D-Trp | $N^\alpha$MeLeu | $Gly\text{-}NH_2$ |
| 30 | H | " | " | " | Leu | " |
| 31 | Ac | Dehydro-D,L-Pro | " | " | " | " |
| 32 | H | " | " | " | $N^\alpha$MeLeu | " |
| 33 | Ac | Dehydro-Pro | " | " | Leu | " |
| 34 | Ac | Dehydro-D-Pro | " | " | " | " |
| 35 | H | Dehydro-Pro | " | " | " | " |
| 36 | H | Dehydro-D-Pro | " | " | " | " |
| 37 | H | Dehydro-Pro | " | " | " | $NHCH_2CH_3$ |
| 38 | Bz | Dehydro-Pro | D-Trp | " | " | $Gly\text{-}NH_2$ |
| 39 | For | Dehydro-Pro | " | D-Phe | " | " |
| 40 | For | Dehydro-Pro | D-His | Trp | " | " |
| 41 | Acr | Dehdyro-Pro | " | " | " | " |
| 42 | Bz | Dehydro-Pro | Trp | D-His | " | " |
| 43 | Acr | Dehydro-Pro | " | D-Trp | " | " |
| 44 | For | Dehydro-Pro | " | D-His | " | " |
| 45 | Bz | Dehydro-Pro | D-His | " | " | " |

14. Verfahren zum Herstellen einer Verbindung der allgemeinen Formel

$$X\text{-}R_1\text{-}R_2\text{-}R_3\text{-Ser-Tyr-}R_4\text{-}R_5\text{-Arg-Pro-}R_6$$

worin X für Wasserstoff oder eine Acylgruppe mit 7 oder weniger Kohlenstoffatomen, $R_1$ für Dehydro-Pro, Dehydro-D-Pro, Thz oder D-Thz, $R_2$ für D-Phe, D-His, D-Trp, Trp, Cl-D-Phe, Dichlor-D-Phe, $CF_3$-D-Phe, F-D-Phe, Difluor-D-Phe, AcNH-D-Phe, $NO_2$-D-Phe, Dinitro-D-Phe, Br-D-Phe, Dibrom-D-Phe, $CH_3S$-D-Phe, $OCH_3$-D-Phe oder $CH_3$-D-Phe, $R_3$ für D-Trp, Trp, D-Phe oder D-His, $R_4$ für Gly oder eine D-isomere Aminosäure, $R_5$ für Leu oder $N^\alpha$Me-Leu und $R_6$ für $Gly\text{-}NH_2$ oder $NHCH_2CH_3$ steht, dadurch gekennzeichnet, daß

(a) ein Zwischenprodukt der Formel:

$$X^1\text{-}R_1\text{-}R_2\text{-}R_3\text{-Ser}(X^2)\text{-Tyr-}(X^3)\text{-}R_4\text{-}R_5\text{-Arg}(X^4)\text{-Pro-}X^5$$

worin $X^1$ eine Shutzgruppe für eine $\alpha$-Aminogruppe, $X^2$ eine Schutzgruppe für die alkoholische Hydroxylgruppe von Ser, $X^3$ eine Schutzgruppe für die phenolische Hydroxylgruppe von Tyr, $X^4$ eine

Schutzgruppe für die Stickstoffatome von Arg darstellt und $X^5$ aus Gly-O-$CH_2$ (Trägerharz), O-$CH_2$-(Trägerharz), Gly-NH-(Trägerharz), Gly-$NH_2$ und $NHCH_2CH_3$ ausgewählt ist, hergestellt wird, daß

(b) eine oder mehrere der Gruppen $X^1$ bis $X^5$ abgespalten werden und daß gewünschtenfalls ein erhaltenes Peptid in ein nicht-toxisches Salz übergeführt wird.

15. Eine pharmazeutische Mischung, welche eine Peptid der in irgendeinem der Ansprüche 1 bis 13 beanspruchten Art zusammen mit einem Träger oder einem Verdünnungsmittel enthält, welcher bzw. welches die Mischung intravenös, subcutan, intramusculär, oral, intranasal oder intravaginal verabreichbar macht.

**Patentansprüche für den Vertragsstaat AT:**

1. Verfahren zum Herstellen einer Verbindung der allgemeinen Formel

$$X\text{-}R_1\text{-}R_2\text{-}R_3\text{-Ser-Tyr-}R_4\text{-}R_5\text{-Arg-Pro-}R_6$$

worin X für Wasserstoff oder eine Acylgruppe mit 7 oder weniger Kohlenstoffatomen, $R_1$ für Dehydro-Pro, Dehydro-D-Pro, Thz oder D-Thz, $R_2$ für D-Phe, D-His, D-Trp, Trp, Cl-D-Phe, Dichlor-D-Phe, $CF_3$-D-Phe, F-D-Phe, Difluor-D-Phe, AcNH-D-Phe, $NO_2$-D-Phe, Dinitro-D-Phe, Br-D-Phe, Dibrom-D-Phe, $CH_3S$-D-Phe, $OCH_3$-D-Phe oder $CH_3$-D-Phe, $R_3$ für D-Trp, Trp, D-Phe oder D-His, $R_4$ für Gly oder eine D-isomere Aminosäure, $R_5$ für Leu oder $N^{\alpha}$Me-Leu und $R_6$ für Gly-$NH_2$ oder $NHCH_2CH_3$ steht, dadurch gekennzeichnet, daß

(a) ein Zwischenprodukt der Formel:

$$X^1\text{-}R_1\text{-}R_2\text{-}R_3\text{-Ser}(X^2)\text{-Tyr-}(X^3)\text{-}R_4\text{-}R_5\text{-Arg}(X^4)\text{-Pro-}X^5$$

worin $X^1$ eine Schutzgruppe für eine $\alpha$-Aminogruppe, $X^2$ eine Schutzgruppe für die alkoholische Hydroxylgruppe von Ser, $X^3$ eine Schutzgruppe für die phenolische Hydroxylgruppe von Tyr, $X^4$ eine Schutzgruppe für die Stickstoffatome von Arg darstellt und $X^5$ aus Gly-O-$CH_2$ (Trägerharz), O-$CH_2$-(Trägerharz), Gly-NH-(Trägerharz), Gly-$NH_2$ und $NHCH_2CH_3$ ausgewählt ist hergestellt wird, daß

(b) eine oder mehrere der Gruppen $X^1$ bis $X^5$ abgespalten werden und daß gewünschtenfalls ein erhaltenes Peptid in ein nicht-toxisches Salz übergeführt wird.

2. Verfahren nach Anspruch 1, worin $R_2$ für Dichlor-D-Phe, 4-$CF_3$-D-Phe, 4-F-D-Phe, Difluor-D-Phe, 4-AcNH-D-Phe, 4-$NO_2$-D-Phe, Dinitro-D-Phe, 4-Br-D-Phe, Dibrom-D-Phe, 4-$CH_3S$-D-Phe, 4-$OCH_3$-D-Phe oder 4-$CH_3$-D-Phe steht.

3. Verfahren nach Anspruch 1 oder 2, worin $R_1$ für Dehydro-Pro steht.

4. Verfahren nach irgendeinem der Ansprüche 1, 2 und 3, worin X für Acrylyl steht.

5. Verfahren nach irgendeinem der Ansprüche 1, 2 und 3, worin X für Acetyl steht.

6. Verfahren nach irgendeinem der Ansprüche 1 bis 5, worin $R_3$ für D-Trp steht.

7. Verfahren nach irgendeinem der Ansprüche 1 bis 6, worin $R_4$ für D-Trp steht.

8. Verfahren nach irgendeinem der Ansprüche 1 bis 6, worin $R_4$ für eine lipophile, aromatische D-isomere Aminosäure steht.

9. Verfahren nach irgendeinem der Ansprüche 1 bis 8, worin $R_5$ für Leu steht.

10. Verfahren nach irgendeinem der Ansprüche 1 bis 9, worin $R_6$ für Gly-$NH_2$ steht.

**Revendications pour les Etats Contractants: BE CH DE FR GB IT LI LU NL SE**

1. Un peptide (ou un de ses sels non toxiques), ledit peptide ayant pour formule:

$$X\text{-}R_1\text{-}R_2\text{-}R_3\text{-Sér-Tyr-}R_4\text{-}R_5\text{-Arg-Pro-}R_6$$

où X est un hydrogène ou un groupe acyle ayant 7 atomes de carbone ou moins; $R_1$ est déhydro-Pro, déhydro-D-Pro, Thz ou D-Thz; $R_2$ est D-Phé, D-His, D-Trp, Trp, Cl-D-Phé, dichloro-D-Phé, $CF_3$-D-Phé, F-D-Phé, difluoro-D-Phé, AcNH-D-Phé, $NO_2$-D-Phé, dinitro-D-Phé, Br-D-Phé, dibromo-D-Phé, $CH_3S$-D-Phé, $OCH_3$-D-Phé ou $CH_3$-D-Phé; $R_3$ est D-Trp, Trp, D-Phé ou D-His; $R_4$ est Gly ou un isomère D d'amino-acide; $R^5$ est Leu ou $N^{\alpha}$Mé-Leu; et $R_6$ est Gly-$NH_2$ ou $NHCH_2CH_3$.

2. Un peptide selon la revendication 1 dans lequel $R_2$ est dichloro-D-Phé, 4 $CF_3$-D-Phé, 4 F-D-Phé, difluoro-D-Phé, 4 AcNH-D-Phé, 4 $NO_2$-D-Phé, dinitro-D-Phé, 4 Br-D-Phé, dibromo-D-Phé, 4 $CH_3S$-D-Phé, 4 $OCH_3$-D-Phé ou 4 $CH_3$-D-Phé.

3. Un peptide selon l'une des revendications 1 et 2 où $R_1$ est déhydro-Pro.

4. Un peptide selon l'une quelconque des revendications 1, 2 et 3 dans lequel X est un acrylyle.

5. Un peptide selon l'une quelconque des revendications 1, 2 et 3 dans lequel X est un acétyle.

6. Un peptide selon l'une quelconque des revendications 1 à 5, dans lequel $R_3$ est D-Trp.

7. Un peptide selon l'une quelconque des revendications 1 à 6 dans lequel $R_4$ est D-Trp.

8. Un peptide selon l'une quelconque des revendications 1 à 6 dans lequel $R_4$ est un isomère D d'amino-acide aromatique lipophile.

9. Un peptide selon l'une quelconque des revendications 1 à 8 dans lequel $R_5$ est Leu.

10. Un peptide selon l'une quelconque des revendications 1 à 9, où $R_6$ est Gly-$NH_2$.

11. Un peptide tel qu'énuméré (peptides 1—14) dans le tableau ci-dessous et défini par la formule générale suivante:

X-déhydro-Pro-$R_2$-D-Trp-Sér-Tyr-D-Trp-$R_5$-Arg-Pro-Gly-$NH_2$

| Peptide | X | $R_2$ | $R_5$ |
|---|---|---|---|
| 1 | Ac | 3,4 $Cl_2$-D-Phé | Leu |
| 2 | " | 4 $CF_3$-D-Phé | " |
| 3 | " | 4F-D-Phé | " |
| 4 | " | 4 AcNH-D-Phé | " |
| 5 | " | 4 $NO_2$-D-Phé | " |
| 6 | " | 4 Br-D-Phé | " |
| 7 | " | 4 $CH_3S$-D-Phé | " |
| 8 | " | 4 $OCH_3$-D-Phé | " |
| 9 | " | 4 $CH_3$-D-Phé | " |
| 10 | " | 2,4 $Cl_2$-D-Phé | " |
| 11 | Acr | 3,4 $Cl_2$-D-Phé | " |
| 12 | Ac | 4 $OCH_3$-D-Phé | $N^{\alpha}$MéLeu |
| 13 | " | 4 $CH_3$-D-Phé | " |
| 14 | " | 3,4 $Cl_2$-D-Phé | " |

12. Un peptide tel qu'énuméré (peptides 15—28) dans le tableau ci-dessous et défini par la formule générale suivante:

X-$R_1$-p-Cl-D-Phé-D-Trp-Sér-Tyr-$R_4$-$R_5$-Arg-Pro-$R_6$

| Peptide | X | $R_1$ | $R_4$ | $R_5$ | $R_6$ |
|---|---|---|---|---|---|
| 15 | Ac | déhydro Pro | D-Trp | Leu | Gly-$NH_2$ |
| 16 | " | " | " | $N^{\alpha}$MéLeu | " |
| 17 | " | déhydro D-Pro | " | Leu | " |
| 18 | " | " | " | $N^{\alpha}$MéLeu | " |
| 19 | Acr | déhydro Pro | " | " | " |
| 20 | Ac | déhydro D-Pro | " | Leu | $NHCH_2CH_3$ |
| 21 | Ac | Thz | D-Trp | Leu | Gly-$NH_2$ |
| 22 | " | " | " | $N^{\alpha}$MéLeu | " |
| 23 | " | D-Thz | " | " | " |
| 24 | " | " | " | Leu | " |
| 25 | " | déhydro Pro | (imBzl)D-His | " | " |
| 26 | " | déhydro D-Pro | " | " | " |
| 27 | " | Thz | " | " | " |
| 28 | " | D-Thz | " | " | " |

13. Un peptide tel qu'énuméré (peptides 29—45) dans le tableau ci-dessous et défini par la formule générale suivante:

X-$R_1$-$R_2$-$R_3$-Sér-Tyr-D-Trp-$R_5$-Arg-Pro-$R_6$

| Peptide | X | $R_1$ | $R_2$ | $R_3$ | $R_5$ | $R_6$ |
|---|---|---|---|---|---|---|
| 29 | H | déhydro-D,L-Pro | D-Phé | D-Trp | $N^{\alpha}$MéLeu | Gly-$NH_2$ |
| 30 | H | '' | '' | '' | Leu | '' |
| 31 | Ac | déhydro-D,L-Pro | '' | '' | '' | '' |
| 32 | H | '' | '' | '' | $N^{\alpha}$MéLeu | '' |
| 33 | Ac | déhydro-Pro | '' | '' | Leu | '' |
| 34 | Ac | déhydro-D-Pro | '' | '' | '' | '' |
| 35 | H | déhydro-Pro | '' | '' | '' | '' |
| 36 | H | déhydro-D-Pro | '' | '' | '' | '' |
| 37 | H | déhydro-Pro | '' | '' | '' | $NHCH_2CH_3$ |
| 38 | Bz | déhydro-Pro | D-Trp | '' | '' | Gly-$NH_2$ |
| 39 | For | déhydro-Pro | '' | D-Phé | '' | '' |
| 40 | For | déhydro-Pro | D-His | Trp | '' | '' |
| 41 | Acr | déhydro-Pro | '' | '' | '' | '' |
| 42 | Bz | déhydro-Pro | Trp | D-His | '' | '' |
| 43 | Acr | déhydro-Pro | '' | D-Trp | '' | '' |
| 44 | For | déhydro-Pro | '' | D-His | '' | '' |
| 45 | Bz | déhydro-Pro | D-His | '' | '' | '' |

14. Un procédé pour la fabrication d'un composé ayant pour formule:

$$X\text{-}R_1\text{-}R_2\text{-}R_3\text{-Sér-Tyr-}R_4\text{-}R_5\text{-Arg-Pro-}R_6$$

dans laquelle X est un hydrogène ou un groupe acyle ayant 7 atomes de carbone ou moins; $R_1$ est déhydro-Pro, déhydro-D-Pro, Thz ou D-Thz; $R_2$ est D-Phé, D-His, D-Trp, Trp, Cl-D-Phé, dichloro-D-Phé, $CF_3$-D-Phé, F-D-Phé, difluoro-D-Phé, AcNH-D-Phé, $NO_2$-D-Phé, dinitro-D-Phé, Br-D-Phé, dibromo-D-Phé, $CH_3S$-D-Phé, $OCH_3$-D-Phé ou $CH_3$-D-Phé; $R_3$ est D-Trp, Trp, D-Phé ou D-His; $R_4$ est Gly ou un isomère D d'amino-acide; $R_5$ est Leu ou $N^{\alpha}$Mé-Leu; et $R_6$ est Gly-$NH_2$ ou $NHCH_2CH_3$, le procédé comprenant:

(a) la formation d'un composé intermédiaire ayant pour formule:

$$X^1\text{-}R_1\text{-}R_2\text{-}R_3\text{-Sér}(X^2)\text{-Tyr-}(X^3)\text{-}R_4\text{-}R_5\text{-Arg}(X^4)\text{-Pro-}X^5$$

où $X_1$ est un groupe $\alpha$-amino-protecteur $X^2$ est un groupe protecteur du groupe hydroxy alcoolique de Sér: $X^3$ est un groupe protecteur du groupe hydroxy phénolique de Tyr; $X_4$ est un groupe protecteur des atomes d'azote d'Arg; et $X^5$ est choisi parmi Gly-O-$CH_2$-(résine support), O-$CH_2$-(résine support), Gly-NH-(résine support), Gly-$NH_2$ et $NHCH_2CH_3$;

(b) clivage d'un ou plusieurs des groupes $X^1$ à $X^5$ et, si on le désire, conversion d'un peptide obtenu en un de ses sels non toxiques.

15. Une composition pharmaceutique comprenant un peptide tel que revendiqué dans l'une quelconque des revendications 1 à 13, avec un support ou diluant rendant la composition administrable par voie intraveineuse, sous-cutanée, intramusculaire, orale, intranasale ou intravaginale.

**Revendications pour L'etat Contractant AT:**

1. Un procédé pour la fabrication d'un composé ayant pour formule:

$$X\text{-}R_1\text{-}R_2\text{-}R_3\text{-Sér-Tyr-}R_4\text{-}R_5\text{-Arg-Pro-}R_6$$

dans laquelle X est un hydrogène ou un groupe acyle ayant 7 atomes de carbone ou moins; $R_1$ est déhydro-Pro, déhydro-D-Pro, Thz ou D-Thz; $R_2$ est D-Phé, D-His, D-Trp, Trp, Cl-D-Phé, dichloro-D-Phé, $CF_3$-D-Phé, F-D-Phé, difluoro-D-Phé, AcNH-D-Phé, $NO_2$-D-Phé, dinitro-D-Phé, Br-D-Phé, dibromo-D-Phé, $CH_3S$-D-Phé, $OCH_3$-D-Phé ou $CH_3$-D-Phé; $R_3$ est D-Trp, Trp, D-Phé ou D-His; $R_4$ est Gly ou un isomère D d'amino-acide; $R_5$ est Leu ou $N^{\alpha}$MéLeu; et $R_6$ est Gly-$NH_2$ ou $NHCH_2CH_3$, comprenant (a) la formation d'un composé intermédiaire ayant pour formule:

$$X^1\text{-}R_1\text{-}R_2\text{-}R_3\text{-Sér}(X^2)\text{-Tyr-}(X^3)\text{-}R_4\text{-}R_5\text{-Arg}(X^4)\text{-Pro-}X^5$$

où $X^1$ est un groupe $\alpha$-amino protecteur; $X^2$ est un groupe protecteur du groupe hydroxyalcoolique de Sér; $X^3$ est un groupe protecteur du groupe hydroxy phénolique de Tyr; $X^4$ est un groupe protecteur des atomes d'azote d'Arg; et $X^5$ est choisi parmi Gly-O-$CH_2$-[résine support], O-$CH_2$-[résine support], Gly-NH[résine support], Gly-$NH_2$ et $NHCH_2CH_3$;

(b) clivage d'un ou plusieurs des groupes $X^1$ à $X^5$ et si on le désire conversion d'un peptide obtenu en un de ses sels non toxiques.

2. Un procédé selon la revendication 1 dans lequel $R_2$ est dichloro-D-Phé, 4 $CF_3$-D-Phé, 4 F-D-Phé, difluoro-D-Phé, 4 AcNH-D-Phé, 4 $NO_2$-D-Phé, dinitro-D-Phé, 4 Br-D-Phé, dibromo-D-Phé, 4 $CH_3S$-D-Phé, 4 $OCH_3$-D-Phé ou 4 $CH_3$-D-Phé.

3. Un procédé selon l'une des revendications 1 et 2, dans lequel $R^1$ est déhydro-Pro.

4. Un procédé selon l'une quelconque des revendications 1, 2 et 3 dans lequel X est un acrylyle.

5. Un procédé selon l'une quelconque des revendications 1, 2 et 3 dans lequel X est un acétyle.

6. Un procédé selon l'une quelconque des revendications 1 à 5 dans lequel $R^3$ est D-Trp.

7. Un procédé selon l'une quelconque des revendications 1 à 6 dans lequel $R_4$ est D-Trp.

8. Un procédé selon l'une quelconque des revendications 1 à 6, dans lequel $R_4$ est un isomère D d'amino-acide aromatique lipophile.

9. Un procédé selon l'une quelconque des revendications 1 à 8 dans lequel $R_5$ est Leu.

10. Un procédé selon l'une quelconque des revendications 1 à 9 dans lequel $R_6$ est Gly-$NH_2$.